# EUROPEAN PATENT APPLICATION

(11) **EP 4 809 913 A1**
(43) Date of publication of application: **23.09.2026**
(21) Application number: 24891620.7
(22) Date of filing: 06.09.2024
(51) Int. Cl.: A61B 5/026, A61B 5/00, G16H 50/20, G16H 30/20, G16H 30/40

(54) **BLOOD FLOW DATA PREDICTION SYSTEM AND EXECUTION METHOD THEREOF**

(30) Priority: 14.11.2023 KR 20230157364
(71) Applicant: Near Brain Inc., Seoul 06248 (KR)
(72) Inventor: LEE, Tae Rin, Seoul 03068 (KR)
(74) Representative: Nederlandsch Octrooibureau
(86) International application number: PCT/KR2024/013513
(87) International publication number: WO 2025/105673

(57) **Abstract**

A method for predicting blood flow data in a server according to an embodiment of the present disclosure includes generating an AI-based blood flow prediction model, processing a 3D medical image to acquire blood vessel data in the form of a point cloud corresponding to a major blood vessel, specifying an ROI from the blood vessel data and acquiring a blood flow value of a boundary region of the ROI by using the blood flow prediction model, and predicting blood flow data in the ROI by using the blood flow value of the boundary region and the blood vessel data point cloud-shaped corresponding to the ROI.

## Description

### Technical Field

The present disclosure relates to a system and a method for predicting blood flow data, and more particularly, relates to a method for predicting blood flow data at an arbitrary point in a three-dimensional (3D) blood vessel image, and a device thereof.

### Background Art

In modern medicine, a medical image is a crucial tool for the effective diagnosis of diseases and the treatment of patients. Moreover, advances in imaging technology have led to the generation of increasingly sophisticated medical image data. Accordingly, as the volume of data becomes increasingly massive, there are significant difficulties in analyzing medical image data by relying on human vision. Consequently, over the past decade or so, clinical decision support systems and computer-aided diagnosis systems have played an essential role in the automated analysis of medical images.

Conventional clinical decision support systems or computer-aided diagnosis systems perform functions such as detecting and displaying lesion regions or providing interpretive information to medical staff or medical professionals (hereinafter referred to as "users").

For example, Korean Published Patent No. 10-2017-0017614, entitled "Method and Apparatus for Calculating Disease Diagnosis Information Based on Medical Images," discloses a method including steps of detecting a region of interest (ROI) in which an object to be analyzed is captured, calculating a variation coefficient, generating a variation coefficient image, and comparing the image with a reference sample, and further describes the effect of diagnosing the severity of a patient's disease by utilizing medical images obtained through computed tomography devices and ultrasound imaging devices.

Recently, Artificial Intelligence (AI) technologies based on machine learning, including deep learning, have enabled rapid advancements in the analysis and processing of medical images. Deep learning technology applied to medical image is utilized in various tasks such as image analysis, diagnosis, treatment, and prediction. For example, deep learning-based auxiliary diagnostic systems are applied to diagnose cerebrovascular diseases, including cerebral aneurysms.

### Disclosure

### Technical Problem

One objective of the present disclosure is to provide a method for predicting blood flow data. Another objective is to provide a method for generating a 3D blood vessel point image from a medical image and for generating a blood vessel graph including coordinate and structural information of major blood vessels. In addition, the present disclosure aims to provide a system for predicting blood flow data by utilizing a blood vessel graph and the AI model, as well as a method for operating the same. Furthermore, the present disclosure aims to provide a method for predicting blood flow in an ROI within a 3D blood vessel point image.

The objectives of the present disclosure are not limited to those described above, and other objectives and advantages not explicitly mentioned will be apparent from the following description and form embodiments of the present disclosure. In addition, it will be readily understood that the objectives and advantages of the disclosure are achieved by means and combinations set forth in the appended claims. **Technical Solution**

According to an embodiment of the present disclosure, a method for predicting blood flow data in a server includes generating an AI-based blood flow prediction model, processing a 3D medical image to acquire blood vessel data in the form of a point cloud corresponding to a major blood vessel, specifying an ROI from the blood vessel data and acquiring a blood flow value of a boundary region of the ROI by using the blood flow prediction model, and predicting blood flow data in the ROI by using the blood flow value of the boundary region and the blood vessel data in the form of a point cloud corresponding to the ROI.

### Advantageous Effects

According to an embodiment of the present disclosure as described above, blood flow in an ROI may be predicted from a 3D blood vessel point image. In addition, according to an embodiment of the present disclosure, an AI model may be constructed to predict blood flow data based on a blood vessel graph. Furthermore, according to an embodiment of the present disclosure, a blood vessel graph including coordinate and structural information about a major blood vessels may be generated from a 3D blood vessel image, and blood flow data may be calculated by utilizing the blood vessel graph together with the AI model.

### Description of Drawings

FIG. 1 is a diagram for describing a blood flow data prediction system, according to an embodiment of the present disclosure.
FIG. 2 is a schematic diagram illustrating an operation of a blood flow data prediction system, according to an embodiment of the present disclosure.
FIG. 3 is a diagram illustrating an operation of a blood flow data prediction system, according to an embodiment of the present disclosure.
FIG. 4 is a flowchart for describing an embodiment of a 3D medical image processing method, according to an embodiment of the present disclosure.
FIGS. 5 to 8 are example diagrams for describing an operation of a 3D medical image processing device, according to an embodiment of the present disclosure.
FIG. 9 is a flowchart for describing an embodiment of a blood vessel graph generation method, according to an embodiment of the present disclosure.
FIGS. 10 to 14 are example diagrams for describing an operation of a blood vessel graph generation device, according to an embodiment of the present disclosure.
FIG. 15 is a flowchart for describing an embodiment of a blood vessel graph generation method, according to another embodiment of the present disclosure.
FIG. 16 is an example diagram for describing an architecture of a blood vessel graph generation model, according to an embodiment of the present disclosure.
FIG. 17 is a diagram for describing a training method of an AI model for blood flow prediction, according to an embodiment of the present disclosure.
FIGS. 18 and 19 are diagrams for describing an architecture and an operation of an AI model for blood flow prediction, according to an embodiment of the present disclosure.
FIG. 20 is a block diagram for describing a configuration of a server for predicting a blood flow, according to an embodiment of the present disclosure.

### Best Mode

The aforementioned objectives, features, and advantages will be described in detail below with reference to the accompanying drawing, and accordingly, those skilled in the art to which the present disclosure pertains may readily implement the technical concept of the present disclosure. In describing the present disclosure, when detailed descriptions of prior art related to the present disclosure is determined to be deemed to unnecessarily obscure the essence of the present disclosure, the detailed descriptions are omitted. Hereinafter, a preferred embodiment according to the present disclosure will be described in detail with reference to the accompanying drawings. In the drawings, the same reference numerals are used to indicate the same or similar components.

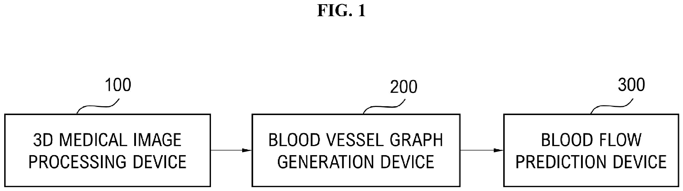

FIG. 1 is a diagram for describing a blood flow prediction system, according to an embodiment of the present disclosure.

Referring to FIG. 1, a blood flow prediction system comprises a 3D medical image processing device 100, a blood vessel graph generation device 200, and a blood flow prediction device 300. Although FIG. 1 illustrates the blood flow prediction system as including a plurality of devices, such illustration is merely for convenience of explanation, and the present disclosure should not be constructed as being limited thereto. That is, in a blood flow prediction system according to an embodiment of the present disclosure, a module performing each function may be executed in a single device, multiple devices, or in a virtualized environment.

The 3D medical image processing device 100 may generate a 3D blood vessel image for a major blood vessels from a 3D medical image.

Upon receiving a 3D medical image, the 3D medical image processing device 100 may analyze pixel values of the 3D medical image to extract blood vessel pixel points and corresponding coordinates. For example, when an arbitrary pixel value in the 3D medical image is greater than or equal to a predetermined threshold value, the 3D medical image processing device 100 may determine that the pixel corresponds to a blood vessel, may designate the pixel as a blood vessel pixel point, and may extract coordinate information associated with the blood vessel pixel point.

In an embodiment of the present disclosure, when a pixel value is greater than or equal to a predetermined threshold value, the 3D medical image processing device 100 may extract the coordinates of the pixel and may generate a blood vessel pixel coordinate table based on the extracted pixel coordinates. Subsequently, the 3D medical image processing device 100 may construct a coordinate system of the entire blood vessel by mapping blood vessel points onto the coordinate system based on the blood vessel pixel coordinate table.

The 3D medical image processing device 100 may crop a major blood vessel portion from the blood vessel pixel points mapped on the coordinate system.

Subsequently, the 3D medical image processing device 100 may cluster the blood vessel pixel points and may filter out noise and/or micro-vessel pixels.

In an embodiment of the present disclosure, the 3D medical image processing device 100 may group blood vessel pixel points into clusters by performing clustering based on at least one of distance, density, or attribute information of the blood vessel pixel points.

Subsequently, the 3D medical image processing device 100 may perform a first filtering operation by comparing the number of blood vessel pixel points included in an arbitrary cluster with a threshold value, and deleting clusters in which the number of pixel points is less than or equal to the threshold value. The filtering may be performed by removing pixel points corresponding to such clusters from the blood vessel coordinate system. In this way, noise around the major blood vessels may be removed.

The 3D medical image processing device 100 may group blood vessel pixel points into clusters by performing clustering again on the first-filtered blood vessel pixel points, based on at least one of distance, density, or attribute information.

Subsequently, the 3D medical image processing device 100 may perform a second filtering operation by excluding blood vessel pixel points included in an arbitrary cluster and deleting the remaining clusters. For example, the filtering may be performed by removing pixel points corresponding to clusters other than the largest cluster from the blood vessel coordinate system. In this way, micro-vessels may be removed.

According to another embodiment of the present disclosure, the 3D medical image processing device 100 may perform clustering and/or filtering of blood vessel pixel points using various algorithms, and the present disclosure should not be construed as limiting the clustering and/or filtering algorithms.

For example, the clustering may be performed by grouping blood vessel pixel points into K clusters, calculating the center of each cluster, and assigning each blood vessel pixel point to the cluster with the nearest center. As another example, clusters may be formed using a similarity matrix for blood vessel pixel points. Afterwards, filtering may be performed on micro-vessels and noise around blood vessels by modifying or removing clusters.

According to an embodiment of the present disclosure, when the 3D medical image processing device 100 performs filtering through clustering, blood vessel pixel points with noise and micro-vessels removed may be arranged in the blood vessel coordinate system, and the 3D medical image processing device 100 may thereby generate a 3D blood vessel point image.

The blood vessel graph generation device 200 may generate a blood vessel graph based on the 3D blood vessel image produced by the 3D medical image processing device 100.

The blood vessel graph generation device 200 may divide a 3D blood vessel image into a plurality of layers and may identify the coordinate values of blood vessel pixel points for each layer. In this case, it is appropriate to generate the layers in two dimensions.

Subsequently, the blood vessel graph generation device 200 may group blood vessel pixel points within each layer. The group of blood vessel pixel points of each layer may correspond to a cross-section of the actual blood vessel.

Subsequently, the blood vessel graph generation device 200 may calculate the centroid for each group. This process is to generate nodes of the blood vessel graph, wherein the nodes of the blood vessel graph according to an embodiment of the present disclosure may be defined as the centroid of the group of blood vessel pixel points for each layer. In the blood vessel graph according to an embodiment of the present disclosure, a node may correspond to the center of the actual blood vessel, and it is appropriate to set the radius of the node of the blood vessel graph to the radius of the actual blood vessel.

Subsequently, the blood vessel graph generation device 200 may generate connectivity information for nodes of the blood vessel graph. More specifically, the blood vessel graph generation device 200 may generate connectivity information between nodes of a first group and nodes of a second group based on whether an intersection exists between the blood vessel pixel points of the first group in an arbitrary layer and those of the second group in another layer adjacent to the layer. The group of blood vessel pixel points in each layer corresponds to a cross-section of an actual blood vessel, and when an intersection with an adjacent layer is present, such intersection is interpreted as representing an actual blood vessel connection.

In this manner, the blood vessel graph generation device 200 may generate nodes and connectivity information from a 3D blood vessel image and thereby construct a blood vessel graph. In the case, the blood vessel graph may be generated as a one-dimensional (1D) network. Furthermore, the blood vessel graph generation device 200 may calculate the actual radius of the blood vessel in the blood vessel graph and may set the radius of each node in the blood vessel graph accordingly. In this way, characteristics such as branching, connection, size, and location of the blood vessel may be reflected in the blood vessel graph generated according to an embodiment of the present disclosure. A specific method for this is described later with reference to the accompanying drawings.

According to another embodiment of the present disclosure, the blood vessel graph generation device 200 may generate the blood vessel graph by extracting a skeleton from a 3D blood vessel image.

To this end, according to an embodiment of the present disclosure, the blood vessel graph generation device 200 may generate an AI model configured to produce a blood vessel graph by extracting the skeleton from the 3D blood vessel image. The AI model may be trained to extract the skeleton in which features of blood vessel pixel points in the 3D blood vessel image are compressed. The training method and architecture of the AI model may vary, and the present disclosure should not be construed as being limited thereto.

For example, the blood vessel graph generation device 200 may be configured such that the AI model for generating the blood vessel graph includes an encoder and a decoder. The encoder may extract features by down-sampling the 3D blood vessel image data, and the decoder may restore the skeleton by using the extracted features. Furthermore, the AI model may be configured to transfer information from each layer of the encoder to the corresponding layer of the decoder. This is intended to restore spatial information of the image while preserving feature information of the 3D blood vessel image.

Furthermore, to train the AI model, the blood vessel graph generation device 200 may set labels or masks that represent the skeleton in the 3D blood vessel image data and use them as training data for the AI model. In addition, the blood vessel graph generation device 200 may adjust the number of input and output channels of the AI model to match the training data and may define an appropriate loss function. For example, when a BCEWithLogitsLoss function is employed as the loss function, the model may convert an input value into a probability value and apply a Binary Cross-Entropy Loss function.

Subsequently, the blood vessel graph generation device 200 may input the 3D blood vessel image data into the AI model and may update weights of the model to minimize a loss between the desired output (skeleton) and the actual output. Furthermore, the blood vessel graph generation device 200 may train the blood vessel graph generation model by evaluating the accuracy of the training data and monitoring it's performance using validation data.

Upton completion of training of the AI model for generating the blood vessel graph, the blood vessel graph generation device 200 may extract a skeleton from a 3D blood vessel image using the model and thereby generate the blood vessel graph.

The blood flow prediction device 300 may predict the blood flow of an ROI based on a 3D blood vessel point image.

For example, the blood flow prediction device 300 may enable the 3D blood vessel point image generated by the 3D medical image processing device 100 to be displayed on a user terminal and may designate an arbitrary region of the 3D blood vessel point image as an ROI. Subsequently, the blood flow prediction device 300 may calculate a blood flow prediction value, such as velocity or pressure of blood flow, from 3D blood vessel point image data of the ROI by applying Computational Fluid Dynamics (CFD) or Computer-Aided Engineering (CAE). In this case, the blood flow prediction device 300 needs to apply blood flow data of a boundary region of the ROI as a boundary condition.

To this end, the blood flow prediction device 300 may calculate the blood flow prediction value by using a blood vessel graph and interpolate the value into 3D blood vessel point image data in the form of a point cloud, thereby obtaining data related to the velocity and/or pressure of the blood flow in the boundary region.

In more detail, the blood flow prediction device 300 may obtain a blood vessel graph representing the shape of the blood vessel from coordinate information corresponding to the ROI from the blood vessel graph generation device 200. Furthermore, the blood flow prediction device 300 may generate a blood flow prediction model configured to predict the pressure of an arbitrary node by using location and radius information of each node in the blood vessel graph. A training method for the blood flow prediction model according to an embodiment of the present disclosure will be described later together with the reference to the accompanying drawings.

When the blood flow prediction model is prepared, the blood flow prediction device 300 may distinguish between a boundary node located at the end of the blood vessel graph and another node located inside, and may acquire a pressure value 'p' of the boundary node together with location information (x, y, z) and node radius 'r' of the boundary node and another node to input this into the model.

The blood flow prediction model may predict the pressure of an arbitrary node (query node) and output the predicted results, and the blood flow prediction device 300 may thereby predict the blood flow at any point in the blood vessel graph.

Subsequently, the blood flow prediction device 300 may apply blood flow data of a node corresponding to the boundary region of the ROI as the boundary condition to CFD or CAE, thereby predicting the blood flow of the ROI based on 3D blood vessel point image data.

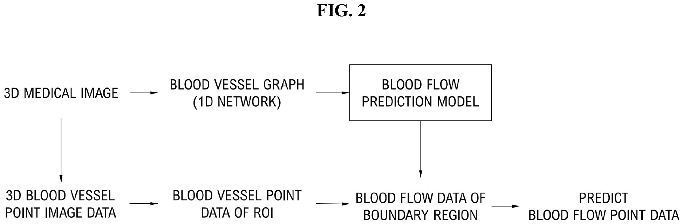

FIG. 2 is a schematic diagram illustrating an operation of a blood flow data prediction system, according to an embodiment of the present disclosure. A blood flow prediction system according to an embodiment of the present disclosure may predict blood flow in an ROI based on 3D blood vessel point data.

In more detail, the blood flow prediction system may generate 3D blood vessel point image data from a 3D medical image and may generate a blood vessel graph using the 3D blood vessel point image data. Subsequently, the blood flow prediction system may predict blood flow data from the blood vessel graph by employing a blood flow prediction model that has been trained.

More specifically, the blood flow prediction system may extract a blood vessel graph from the 3D medical image. The blood vessel graph may be represented in the form of a point cloud and configured as a 1D network connecting points (or nodes). The graph may include coordinate information composed of x, y, and z values of nodes constituting a blood vessel, and the radius 'r' of a node corresponding to the actual blood vessel radius.

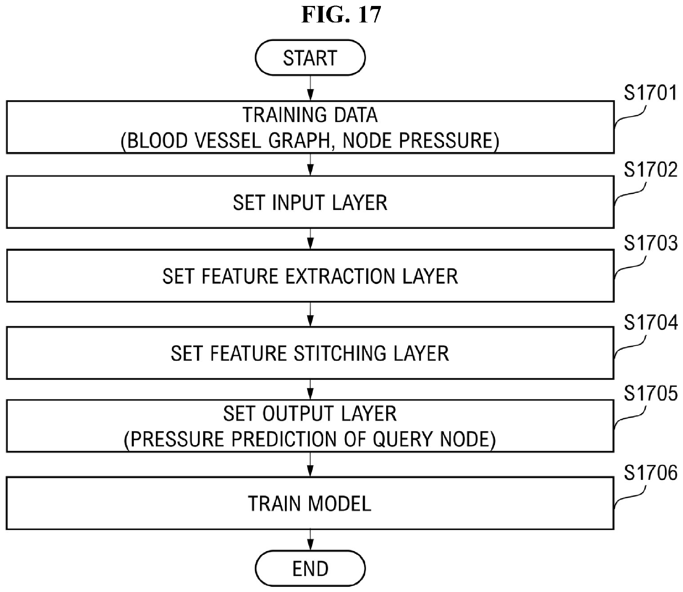

Furthermore, the blood flow prediction system may allow the blood vessel graph to be input into the blood flow prediction model that has been trained. The training method and architecture of the blood flow prediction model will be described in more detail later with reference to attached FIGS. 17 to 19.

The blood flow prediction system may predict the blood flow at any point in the blood vessel graph, which is represented as a 1D network, by using the blood flow prediction model to calculate the macroscopic blood flow of the entire blood vessel, such as velocity and/or pressure. More specifically, the blood flow prediction device 300 may distinguish between a boundary node located at the end of the blood vessel graph and another node located inside, and may acquire the blood flow value of the boundary node together with location information (x, y, z) and a node radius 'r' of the boundary node and another node to input this into the model. The blood flow prediction model may predict and output the pressure of an arbitrary node (query node), and the blood flow prediction device 300 may thereby calculate the macroscopic blood flow of the entire blood vessel by predicting the blood flow of all nodes in the blood vessel graph.

Meanwhile, when an arbitrary region is designated as an ROI in a 3D blood vessel point image, the blood flow prediction system can compute a blood flow prediction value, such as velocity or pressure of a blood flow, from the 3D blood vessel point data by using CFD or CAE.

In this case, the blood flow prediction system may apply blood flow data of the boundary region of the ROI as a boundary condition to CFD or CAF by utilizing the blood vessel graph and the blood flow prediction model. That is, the blood flow prediction system may calculate the total blood flow constituting the blood vessel graph by means of the blood flow prediction model, interpolate the calculated flow to 3D blood vessel point image data in the form of a point cloud, and thereby obtain data related to blood flow velocity and/or blood flow pressure of the boundary region. The obtained data may then be applied as a boundary condition, whereby the blood flow of the ROI may be predicted based on the 3D blood vessel point image data.

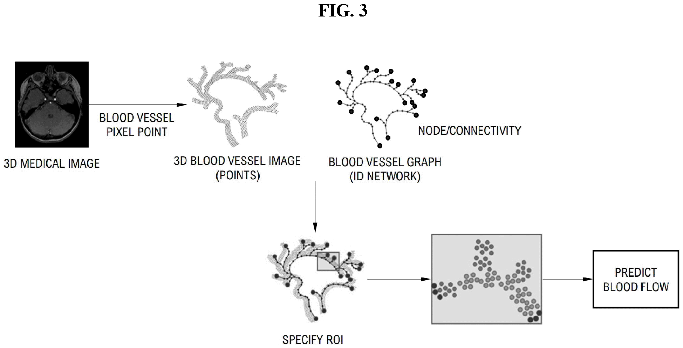

FIG. 3 is a diagram illustrating an operation of a blood flow data prediction system, according to an embodiment of the present disclosure.

A blood flow prediction system according to an embodiment of the present disclosure may acquire a 3D medical image, may extract blood vessel pixel points to generate a 3D blood vessel image, and may generate a blood vessel graph based on the 3D blood vessel image. Subsequently, when an ROI is designated through a user input in the 3D blood vessel image, the blood flow prediction system may compute a blood flow prediction value with respect to the velocity or pressure of the blood flow from the 3D blood vessel point data by means of CFD or CAE.

Furthermore, the blood flow prediction system may acquire blood flow data at a boundary of the ROI and apply the acquired data as a boundary condition. The blood flow data at the ROI boundary may include, for example, blood pressure data at the ROI boundary, which may be a measured value or a prediction value calculated by an algorithm. The prediction value may be calculated by means of the blood vessel graph and a blood flow prediction model.

For example, a blood flow prediction system may convert the ROI into 3D point cloud data, 3D geometry data, or mesh data, and may apply the boundary condition to CFD or CAE in order to predict blood flow data 'P', such as pressure at any query point.

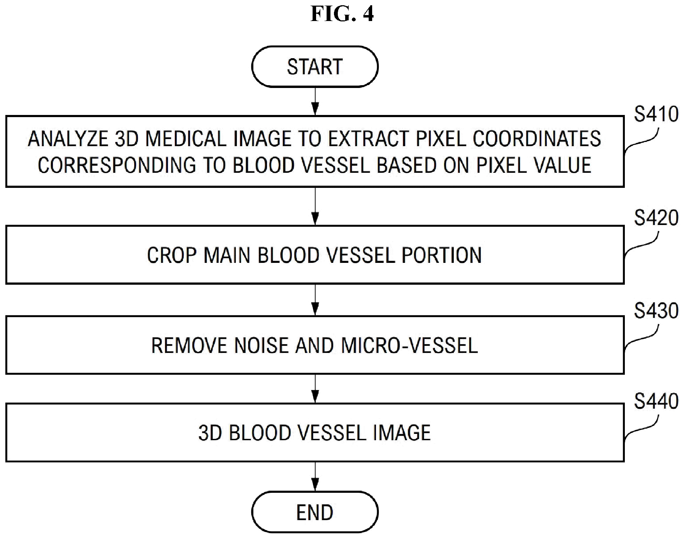

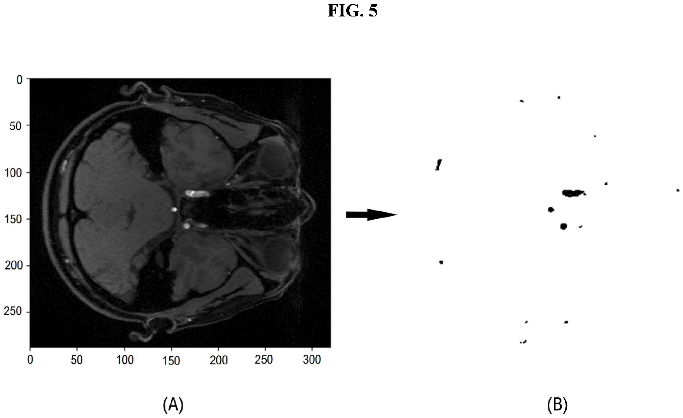

FIG. 4 is a flowchart for describing an embodiment of a 3D medical image processing method, according to an embodiment of the present disclosure. FIGS. 5 to 8 are example diagrams for describing an operation of a 3D medical image processing device, according to an embodiment of the present disclosure.

Referring to FIG. 4, the 3D medical image processing device 100 may analyze a 3D medical image to extract pixel coordinates corresponding to a blood vessel based on pixel values (operation S410).

In an embodiment of operation S410, the 3D medical image processing device 100 may analyze a 3D medical image, may determine a blood vessel when a pixel value is greater than or equal to a predetermined threshold value so as to specify a blood vessel pixel point, and may extract coordinate information of the blood vessel pixel point. Subsequently, the device may map the blood vessel point onto a coordinate system based on the extracted coordinate information, thereby constructing a coordinate system of all blood vessels.

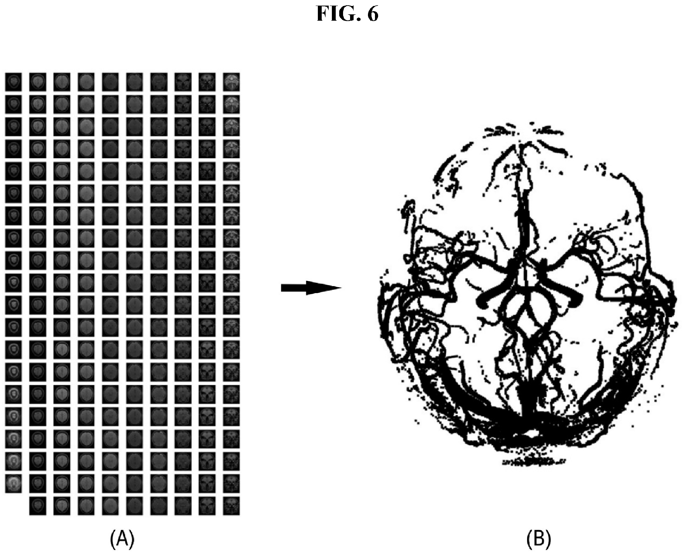

For example, the 3D medical image processing device 100 may analyze the 3D medical image of FIG. 5(A), may extract pixel coordinates corresponding to a blood vessel based on pixel values as shown in FIG. 5(B), and may generate a pixel coordinate table as illustrated in FIG. 6(A). The coordinate system of the entire blood vessel may then be constructed based on the pixel coordinate table, as shown in FIG. 6(B). That is, the coordinate system of the entire blood vessel may be formed by mapping blood vessel pixel points on the coordinate system.

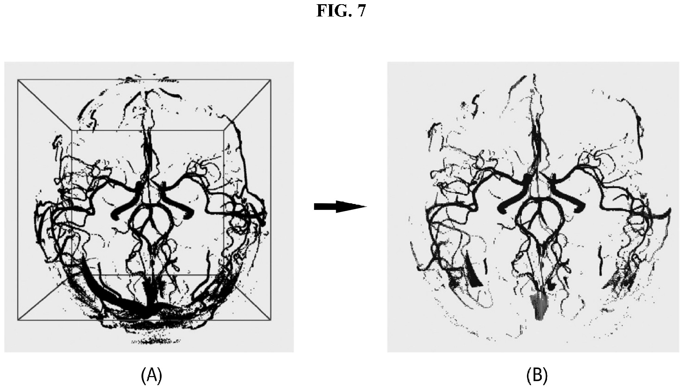

The 3D medical image processing device 100 may crop a major blood vessel portion from the blood vessel pixel points mapped onto the coordinate system (operation S420). For example, the 3D medical image processing device 100 may specify the major blood vessel portion that is the subject of image processing as shown in FIG. 7(B), from among the pixel points of the entire blood vessel displayed on the coordinate system, as shown in FIG. 7(A).

Furthermore, the 3D medical image processing device 100 may remove noise and/or micro-vessel pixels by performing clustering and filtering on blood vessel pixel points within the cropped region (operation S430), and may thereby generate a 3D blood vessel image (operation S440).

For example, the 3D medical image processing device 100 may group blood vessel pixel points into clusters by performing clustering based on at least one of distance, density, or attribute information of the blood vessel pixel points. Subsequently, filtering may be performed by comparing the number of blood vessel points included in each cluster with a threshold value and deleting any cluster having a number of blood vessel points less than or equal to the threshold value. The filtering may be performed by removing pixel points corresponding to such cluster from the blood vessel coordinate system. In this manner, noise around the major blood vessel may be eliminated.

Furthermore, the 3D medical image processing device 100 may group blood vessel pixel points into clusters by performing clustering again on the primarily filtered blood vessel pixel points based on at least one of their distance, density, or attribute information. Thereafter, filtering may be performed by deleting remaining clusters that exclude blood vessel pixel points included in arbitrary clusters. For example, the filtering may be carried out by removing pixel points corresponding to clusters other than the largest cluster from the blood vessel coordinate system. In this manner, micro-vessels may be removed.

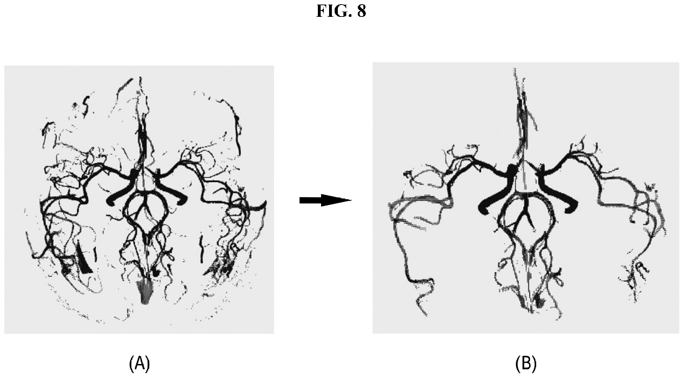

For example, the 3D medical image processing device 100 may generate a 3D blood vessel image, as shown in FIG. 8(B), by removing noise and micro-vessel pixels from a blood vessel to be image-processed, as shown in FIG. 8(A).

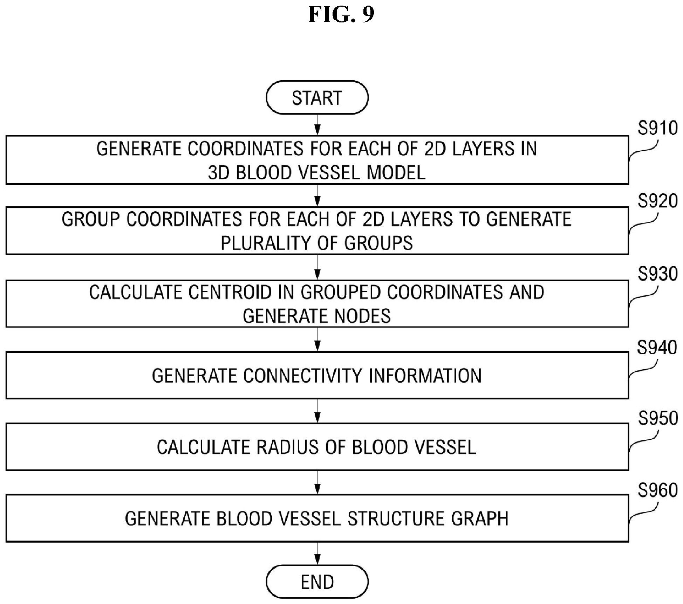

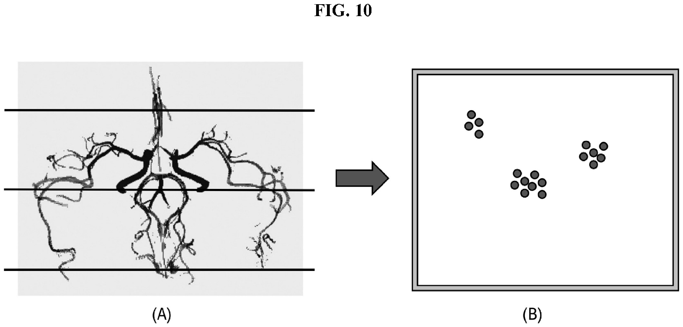

FIG. 9 is a flowchart for describing an embodiment of a blood vessel graph generation method, according to an embodiment of the present disclosure. FIGS. 10 to 14 are example diagrams for describing the execution process of FIG. 9.

Referring to FIG. 9, the blood vessel graph generation device 200 may divide a 3D blood vessel image into multiple layers and identify the coordinate values of blood vessel pixel points for each layer. In this case, it is appropriate to generate the layers in two dimensions (operation S910). For example, the blood vessel graph generation device 200 may divide a 3D blood vessel image into multiple layers, as shown in FIG. 10(A), and may identify the coordinate values of blood vessel pixel points for each layer, as shown in FIG. 10(B).

Afterwards, the blood vessel graph generation device 200 may group the blood vessel pixel points for each layer (operation S920) and may calculate the centroid of each group. This process is to generate nodes of the blood vessel graph, wherein the nodes of the blood vessel graph according to an embodiment of the present disclosure may be defined as the centroid of the group of blood vessel pixel points for each layer (operation S930).

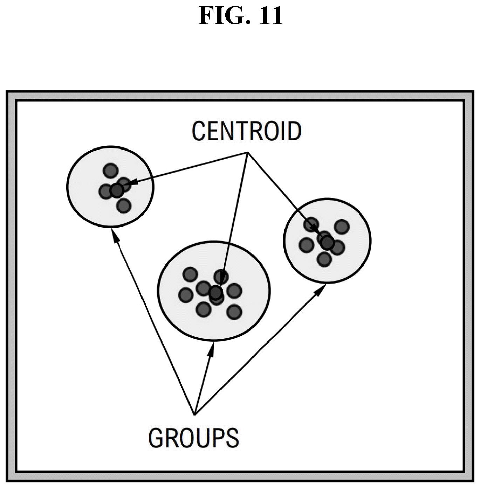

For example, the blood vessel graph generation device 200 may group blood vessel pixel points in any layer, as shown in FIG. 11, and may define the centroid of the group as a node of the blood vessel graph. In the example of FIG. 11, the group of blood vessel pixel points may correspond to a cross-section of an actual blood vessel, and the centroid of the group may correspond to the center of the cross-section of the blood vessel. Accordingly, it is appropriate to set the radius of the node in the blood vessel graph to the radius of the actual blood vessel.

Subsequently, the blood vessel graph generation device 200 may generate connectivity information among the nodes of blood vessel graph (operation S940).

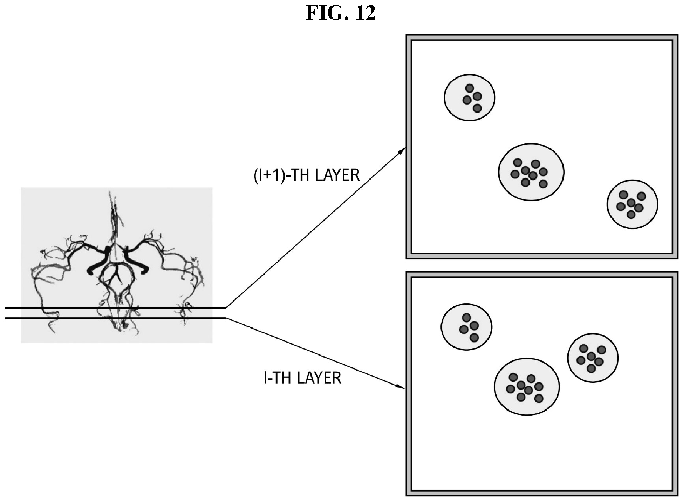

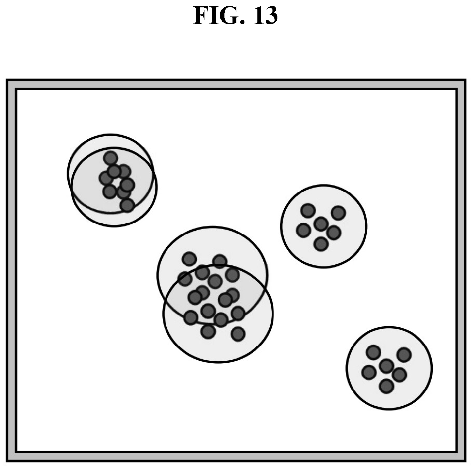

For example, the blood vessel graph generation device 200 may identify the group of blood vessel pixel points of an i-th layer and the group of blood vessel pixel points of the (i+1)-th layer adjacent to the i-th layer, as shown in FIG. 12, and may generate connectivity information in the nodes when an intersection exists between the groups, as shown in FIG. 13. The group of blood vessel pixel points in each layer corresponds to a cross-section of an actual blood vessel, and when an intersection with an adjacent layer is present, such intersection is interpreted as representing an actual blood vessel connection.

Furthermore, the blood vessel graph generation device 200 may calculate the radius of the actual blood vessel in the blood vessel graph and may set it as the node radius (operation S950).

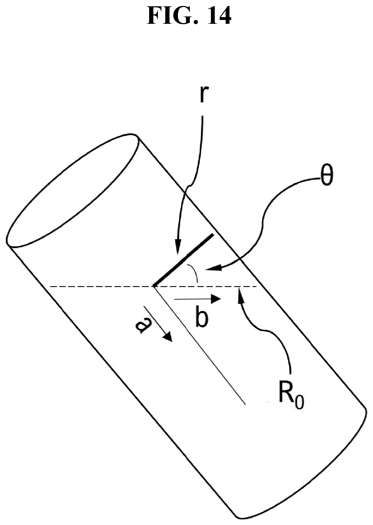

Referring to FIG. 14, when the 3D blood vessel image is divided into 2D layers, the cross-section radius corresponds to a radius R₀ of a blood vessel pixel point group. That is, since R₀ represents the radius of the blood vessel pixel point group rather than the actual radius of the blood vessel, it is necessary to calculate the actual radius 'r' of the blood vessel. To this end, the radius R₀ of the blood vessel pixel point group may be converted by cosθ, and the actual radius 'r' may be calculated according to Equation 1. $r = {R}_{0} \times cos θ$
R₀ denotes the radius of a blood vessel pixel point group;
r denotes the actual radius of the blood vessel; and,
cosθ is calculated according to Equation 2, where θ is calculated by using the connectivity information direction *̅a̅*̅ of the adjacent layer and the radius direction *̅b̅*̅ of the blood vessel pixel point group in the corresponding layer. $cos θ = \frac{\vec{a} ⋅ \vec{b}}{\left|\vec{a}\right| ⋅ \left|\vec{b}\right|}$

*̅a̅*̅ denotes a longitudinal direction calculated from the connectivity information, and *̅b̅*̅ denotes a radial direction of the blood vessel cross-section.

*̅a̅*̅ is perpendicular to the length direction calculated from connectivity information, and *̅b̅*̅ is perpendicular to the blood vessel pixel point group.

Returning to the description of FIG. 9, in operation S960, the blood vessel graph generation device 200 may generate a blood vessel graph by using nodes, connectivity information, and node radii. This graph provides a visual representation of the blood vessel structure, in which information regarding characteristics such as the branching, connectivity, size, and location of the blood vessel is compressed, and parameters such as the length, diameter, angle, branching pattern, and shape of the blood vessel are included.

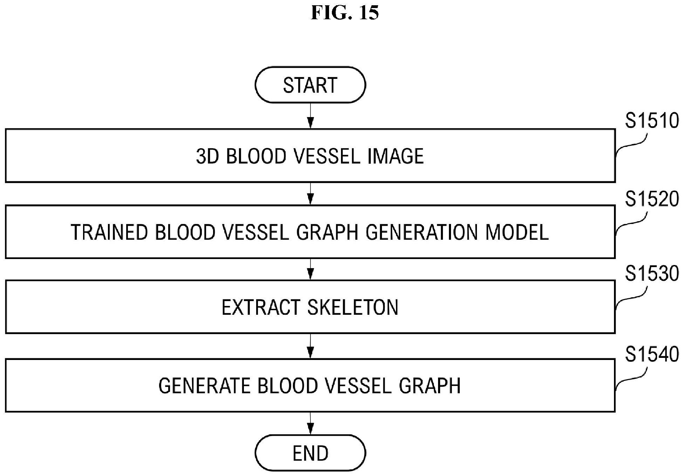

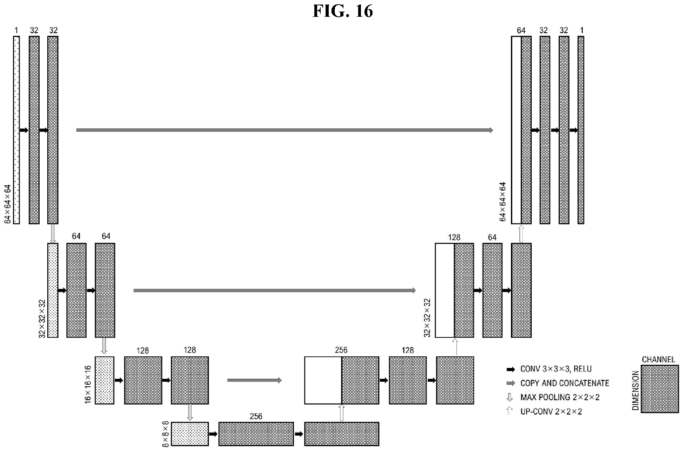

FIG. 15 is a flowchart for describing an embodiment of a blood vessel graph generation method, according to another embodiment of the present disclosure. FIG. 16 is an example diagram for describing an architecture of a blood vessel graph generation model, according to an embodiment of the present disclosure.

According to another embodiment of the present disclosure, the blood vessel graph generation device 200 may acquire a 3D blood vessel image in the form of a point cloud (operation S1510), may extract a skeleton of the 3D blood vessel image (operation S1530) by using a trained blood vessel graph generation model (operation S1520), and may generate a blood vessel graph (operation S1540). For example, the blood vessel graph generation device 200 may generate a blood vessel graph by inputting a 3D blood vessel image of 64×64×64 into a blood vessel graph generation model and extracting a skeleton image of 64×64×64.

In more detail, the blood vessel graph generation device 200 may generate and train an AI model having the architecture illustrated in FIG. 16. The blood vessel graph generation device 200 may train the model, i.e., the blood vessel graph generation model, to extract a skeleton in which the blood vessel pixel point features of a 3D blood vessel image are compressed.

The blood vessel graph generation model may include an encoder and a decoder, as shown in FIG. 16. The encoder may extract features by down-sampling the 3D blood vessel image data, and the decoder may restore the skeleton by using the extracted features. The blood vessel graph generation model may further deliver information from each layer of the encoder to the corresponding layer of the decoder. This is intended to restore spatial information of the image while preserving feature information of the 3D blood vessel image.

The blood vessel graph generation model may be trained using data in which a mask or a label representing the skeleton in the 3D blood vessel image data is set. The number of input and output channels of the blood vessel graph generation model may be adjusted to suit training data, and an appropriate loss function may be defined. For example, when a BCEWithLogitsLoss function is used as the loss function, the model may convert an input value into a probability value and may apply a Binary Cross-Entropy Loss function.

For training the blood vessel graph generation model, when the training data in which a mask or label representing the skeleton in the 3D blood vessel image data is set is input, the weight of the model may be updated to minimize the loss between the correct answer output (skeleton) and the predicted output. Furthermore, the blood vessel graph generation model may be trained by evaluating the accuracy of the training data and monitoring performance using validation data.

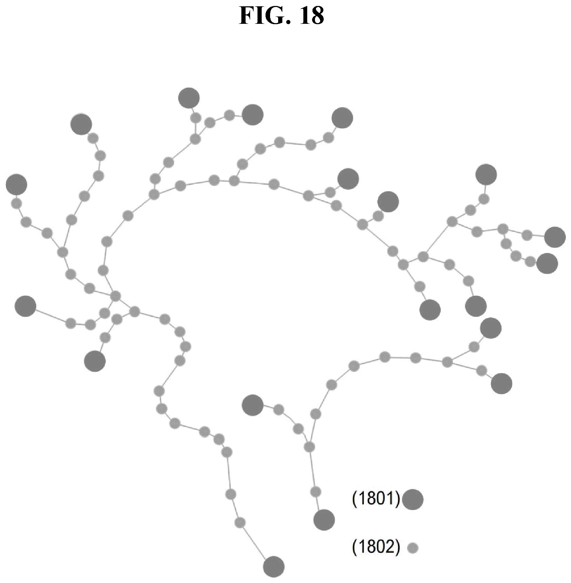

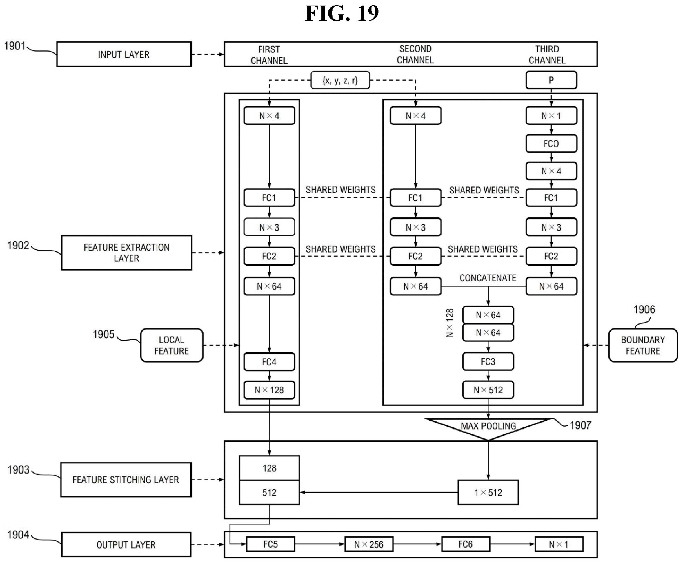

FIG. 17 is a diagram for describing a training method of an AI model for blood flow prediction, according to an embodiment of the present disclosure. FIGS. 18 and 19 are diagrams for describing an architecture and an operation of an AI model for blood flow prediction, according to an embodiment of the present disclosure.

Referring to FIG. 17, a blood flow prediction system may collect data for training an AI model for blood flow prediction (operation S1701).

The training data may be a blood vessel graph for an arbitrary region, as shown in FIG. 18. The blood vessel graph is in the form of a point cloud and may be configured as a 1D network connecting points (or nodes). It may include a boundary node 1801 corresponding to the boundary of the region and another node 1802 that is not the boundary node. Furthermore, the blood vessel graph may include coordinate values composed of the x, y, and z coordinates of each node, together with information regarding the radius 'r' of the node corresponding to the actual blood vessel radius. The training data may further include the pressure 'p' of the boundary node 1801, while the pressure 'p' of the other node may be set as a label or a mask.

Moreover, the blood flow prediction system may set an input layer of the blood flow prediction model (operation S1702).

The input layer of the blood flow prediction model according to an embodiment of the present disclosure may be set to a first channel, a second channel, and a third channel, as shown in 1901 of FIG. 19. The first channel may receive coordinate values and radius information (x, y, z, r) of a query node in the blood vessel graph; the second channel may receive the coordinate values and radius information (x, y, z, r) of a boundary node in the blood vessel graph; and, the third channel may receive the pressure value 'p' of the boundary node.

Furthermore, the blood flow prediction system may set a feature extraction layer of the blood flow prediction model (operation S1703).

The feature extraction layer of the blood flow prediction model according to an embodiment of the present disclosure may correspond to 1902 in FIG. 19.

In particular, the feature extraction layer 1902 may separate the pressure value 'p' of the boundary node input in the third channel into four values corresponding to (x, y, z, r), thereby reflecting the correlation between the node's coordinate values and radius information (x, y, z, r). Furthermore, the feature extraction layer 1902 may pass the coordinate values and radius information (x, y, z, r) input from each channel, or the four separated pressures 'p', through fully connected layers FC1 and FC2 that share weights.

In this way, the feature extraction layer 1902 may form a local feature 1905 that reflects the characteristics of the query node and a boundary feature 1906 that reflects the characteristics of the boundary node. In this case, it is appropriate to apply a max pooling layer 1907 to the boundary feature 1906 to remove input order information of the boundary node.

Furthermore, the blood flow prediction system may set a feature stitching layer (operation S1704).

The feature stitching layer of the blood flow prediction model according to an embodiment of the present disclosure may have an architecture that combines a local feature and a boundary feature and passes them through the fully connected layers FC1 and FC2, as shown in 1903 of FIG. 19. This is intended to enable the model to understand global features across the entire channel.

Furthermore, the blood flow prediction system may set an output layer of the blood flow prediction model (operation S1705). The output layer of the blood flow prediction model according to an embodiment of the present disclosure may be configured to predict and output the pressure of a query node, as shown in 1904 of FIG. 19.

When training data for the blood flow prediction model has been prepared and the structural configuration of the model has been completed, the blood flow prediction system may train the blood flow prediction model (operation S1706).

When the training of the blood flow prediction model has been completed, the blood flow prediction system may specify the blood vessel graph of an ROI, may distinguish between a boundary node located at the end of the specified blood vessel graph and another node located inside, and may acquire the pressure value 'p' of the boundary node together with location information (x, y, z) and the node radius 'r' of the boundary node and another node to input this into the model. The blood flow prediction model may then predict the pressure of an arbitrary node (a query node) and may output the predicted results, thereby enabling the blood flow prediction system to predict the blood flow at an arbitrary point.

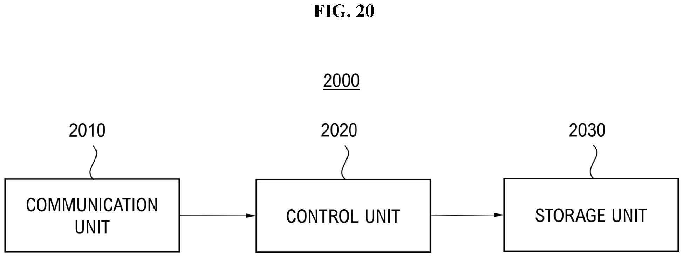

FIG. 20 is a block diagram for describing a configuration of a server for predicting a blood flow, according to an embodiment of the present disclosure.

As illustrated in FIG. 20, a server 2000 according to an embodiment of the present disclosure may include a communication unit 2010, a storage unit 2030, and a control unit 2020, and may further include an input unit and a display unit, although not illustrated in FIG. 20. Although FIG. 20 illustrates that the server 2000 includes the communication unit 2010, the storage unit 2030, and the control unit 2020, each block may be physically separated. For example, the storage unit 2030 may be present in a virtualized data center and may be connected to the control unit 2020 of the server 2000 through the communication unit 2010.

The communication unit 2010 performs the function of transmitting and receiving data for wired and wireless communication of the server 2000, and performs the function of receiving data through a wired or wireless channel, outputting the data to the control unit 2020, and transmitting the data output from the control unit 2020 through a wired channel. In particular, the communication unit 2010 according to an embodiment of the present disclosure may perform the function of receiving 3D medical images.

The storage unit 2030 may store programs and data necessary for the operation of the server 2000, and may be divided into a program region and a data region. The data region of the storage unit 2030 according to an embodiment of the present disclosure may store blood vessel pixel point coordinates, a 3D blood vessel image and a blood vessel graph, as well as blood flow values at any point in the blood vessel, for example, pressure values.

Furthermore, the program region of the storage unit 2030 according to an embodiment of the present disclosure may store a computer program that executes a process of predicting blood flow at an arbitrary point in a blood vessel image on the server The computer program may include a blood flow prediction model that predicts the pressure of an arbitrary node by using information about the location and the radius of each node of a blood vessel graph, and may perform the function of predicting blood flow at an arbitrary point by using the blood flow prediction model.

Furthermore, the program region of the storage unit 2030 according to an embodiment of the present disclosure may store a computer program that executes a process of generating a blood vessel graph on the server. The computer program may perform the function of analyzing a 3D medical image to extract blood vessel pixel point coordinates corresponding to a blood vessel based on pixel values, removing noise around the major blood vessel, and generating a 3D blood vessel image, and the function of dividing the 3D blood vessel image into a plurality of layers, setting a blood vessel pixel point group for each layer as a blood vessel node, and generating connectivity information to construct a blood vessel graph.

The control unit 2020 controls the overall operation of each component of the server 2000. In particular, the control unit 2020 according to an embodiment of the present disclosure may perform the function of predicting a blood flow at an arbitrary point in the 3D blood vessel point image. Furthermore, the control unit 2020 may predict the blood flow at an arbitrary point by using a blood flow prediction model that predicts the pressure of an arbitrary node based on information about the location and the radius of each node in the blood vessel graph. In addition, the control unit 2020 may perform the function of analyzing a 3D medical image to extract blood vessel pixel point coordinates corresponding to a blood vessel based on pixel values, removing noise around the major blood vessel, generating a 3D blood vessel image, dividing the 3D blood vessel image into a plurality of layers, setting a blood vessel pixel point group for each layer as a blood vessel node, and generating connectivity information to construct a blood vessel graph.

Although the present disclosure has been described with reference to specific embodiments and drawings, it is not limited thereto, and those skilled in the art will recognize that various modifications and variations are possible based on the above description. Therefore, the scope of the present disclosure should be determined solely by the claims set forth below, and all equivalent or equivalent variations thereof shall be deemed to fall within the scope of the present disclosure.

## Claims

1. A method for predicting blood flow data in a server, the method comprising:
generating an artificial intelligence (AI)-based blood flow prediction model;
processing a three-dimensional (3D) medical image to acquire blood vessel data in the form of a point cloud corresponding to a major blood vessel;
specifying a region of interest (ROI) from the blood vessel data and acquiring a blood flow value of a boundary region of the ROI by using the blood flow prediction model; and
predicting blood flow data in the ROI by using the blood flow value of the boundary region and the blood vessel data in the form of a point cloud corresponding to the ROI.

2. The method of claim 1, wherein the predicting of the blood flow data in the ROI includes:
predicting the blood flow data by applying Computational Fluid Dynamics (CFD) to blood vessel data in the form of a point cloud corresponding to the ROI; and
interpolating the blood flow value of the boundary region to the blood vessel data.

3. The method of claim 2, wherein the generating of the AI-based blood flow prediction model includes:
extracting node location and radius information from a blood vessel graph for an arbitrary region, and preparing a blood flow value of a boundary node forming a boundary of the region together with the node location and radius information as training data for the blood flow prediction model; and
training the blood flow prediction model by setting a blood flow value of a node other than the boundary node as a label or a mask.

4. The method of claim 3, wherein the acquiring of the blood vessel data includes:
acquiring a 3D blood vessel image in the form of a point cloud from the 3D medical image; and
generating a blood vessel graph by extracting a skeleton of the 3D blood vessel image by using a trained blood vessel graph generation model.

5. The method of claim 4, wherein the generating of the AI-based blood flow prediction model includes:
setting an input layer of the blood flow prediction model to include a channel into which location and radius information of another node and the boundary node of the blood vessel graph are input, and a channel into which the blood flow value of the boundary node is input; and
setting a feature extraction layer of the blood flow prediction model to form a local feature that reflects a characteristic of the other node, and a boundary feature that reflects a characteristic of the boundary node with input order information of the boundary node removed.

6. A blood flow data prediction system comprising:
a 3D medical image processing device configured to analyze a 3D medical image, to extract coordinates of blood vessel pixel point corresponding to a blood vessel based on a pixel value, and to generate a 3D blood vessel image;
a blood vessel graph generation device configured to generate a blood vessel graph from the 3D blood vessel image by using blood vessel pixel point group as a node; and
a blood flow prediction device configured to:
generate an AI-based blood flow prediction model,
specify an ROI in the 3D blood vessel image,
obtain a blood flow value of a boundary region of the ROI by using the blood flow prediction model, and
predict blood flow data in the ROI by using the blood flow value of the boundary region and blood vessel data in the form of a point cloud corresponding to the ROI.

7. A server that predicts blood flow data, the server comprising:
a communication unit configured to receive a 3D medical image; and
a control unit configured to:
generate an AI-based blood flow prediction model,
process the 3D medical image to acquire blood vessel data in the form of a point cloud corresponding to a major blood vessel,
specify a boundary region of the blood vessel data,
acquire a blood flow value of the boundary region,
input the blood vessel data and the blood flow value of the boundary region into the blood flow prediction model, and
acquire a blood flow value of an arbitrary point output through the blood flow prediction model.

8. A computer program stored on a medium to perform processing for predicting blood flow data, the computer program performing:
a function of generating an AI-based blood flow prediction model;
a function of processing a 3D medical image to acquire blood vessel data in the form of a point cloud corresponding to a major blood vessel;
a function of specifying a ROI from the blood vessel data and acquiring a blood flow value of a boundary region of the ROI by using the blood flow prediction model; and
a function of predicting blood flow data in the ROI by using the blood flow value of the boundary region and the blood vessel data in the form of a point clou corresponding to the ROI.
